# FASCICULE DE BREVET EUROPEEN

(11) **EP 1 696 837 B1**
(45) Date de publication et mention de la délivrance du brevet: **12.09.2012**
(21) Numéro de dépôt: 04816429.7
(22) Date de dépôt: 17.12.2004
(51) Int. Cl.: A61F 2/44

(54) **PROTHESE DE DISQUE INTERVERTEBRAL**
BANDSCHEIBENPROTHESE
INTERVERTEBRAL DISC PROSTHESIS

(30) Priorité: 22.12.2003 FR 0315177
(43) Date de publication de la demande: 06.09.2006
(73) Titulaire: COMMISSARIAT ENERGIE ATOMIQUE, 75015 Paris (FR)
(72) Inventeur: AARON, Alain, F-29000 Quimper (FR)
(74) Mandataire: Maureau, Philippe
(86) Numéro de dépôt international: PCT/FR2004/003295
(87) Numéro de publication internationale: WO 2005/065595

(56) Documents cités:
- FR-A- 2 709 949
- FR-A- 2 723 841
- FR-A- 2 734 148

## Description

La présente invention a pour objet une prothèse destinée à remplacer un disque intervertébral endommagé de la colonne vertébrale.

Elle trouve une application dans le domaine de la chirurgie orthopédique.

La colonne vertébrale est constituée par un ensemble de vertèbres superposées reliées les unes aux autres par des disques fibro-cartilagineux, appelés disques intervertébraux. Ces disques intervertébraux ont un rôle fondamental dans la statique et la dynamique de la colonne vertébrale : ils assurent la mobilité des vertèbres entre elles.

Ces disques intervertébraux sont souvent l'objet de désordres relatifs à un tassement de vertèbres, une hernie discale, un déplacement de vertèbres ou encore une dégénérescence arthrosique intervertébrale. Ces désordres sont, bien souvent, la cause de douleurs ou de gènes fonctionnelles rebelles aux traitements médicaux ; dans certains cas, ils peuvent même être invalidants.

Le procédé utilisé pour soulager les patients atteints de ces désordres consiste, généralement, en une intervention chirurgicale. Plusieurs techniques d'intervention chirurgicale sont actuellement connues.

La première technique consiste en une excision discale simple ; la zone endommagée du disque intervertébral est simplement supprimée, ce qui supprime de ce fait la biomécanique normale du disque. Les deux vertèbres adjacentes à ce disque ont alors une mobilité réduite l'une par rapport à l'autre. En outre, une telle excision ne constitue pas un remède définitif au mal du patient, la dégradation du disque pouvant continuer à évoluer.

La seconde technique d'intervention chirurgicale consiste en une arthrodèse intervertébrale par laquelle les deux vertèbres adjacentes au disque endommagé sont fusionnées. Ce procédé est, en effet, un remède définitif qui bloque l'évolution arthrosique, c'est-à-dire la dégradation, dans le disque. Ce procédé a pour principal inconvénient de supprimer toute mobilité discale.

La troisième technique, la plus répandue actuellement, consiste en une chirurgie arthroplastique intervertébrale de remplacement du disque endommagé par une prothèse. De nombreux types de prothèses discales sont connus et décrits dans l'ouvrage intitulé "The Artificial Disc", édité par Mario BROCK, H. Mickae]. MAYER et Kiaus WEIGEL chez SPRINGER-VERLAG, 1991.

Selon cet ouvrage, les prothèses discales connues peuvent être classées en deux grandes catégories :
- Les prothèses pour lesquelles la partie fibreuse du disque naturel est conservée et utilisée comme enveloppe de prothèse. Le noyau intérieur du disque naturel est alors remplacé, soit par un produit polymérisant injecté dans l'enveloppe, soit par des pièces élastiques introduites dans l'enveloppe, soit par un sac inséré dans l'enveloppe et gonflé au moyen d'un liquide ou d'un polymère. Une telle prothèse est décrite, notamment, dans les documents US-A-4 772 287 et EP-A-O 277 282. La difficulté principale, pour réaliser une telle prothèse, réside dans le fait qu'il faille retrouver la géométrie et la résistance en compression du disque naturel. En outre, une telle prothèse ne peut être considérée comme un remède définitif pour le patient, la partie fibreuse conservée du disque pouvant continuer à se dégrader.
- Les prothèses entièrement artificielles qui se substituent à un disque naturel complètement retiré.

Une première technique de réalisation de ces prothèses entièrement artificielles consiste à introduire une articulation glissante entre les deux vertèbres. Cette articulation peut être en contact direct sur les plateaux vertébraux ; elle peut aussi consister en une rotule centrée comme le décrit le document FR-A-2 372 622. Une telle prothèse à rotule centrée a pour inconvénient que le déplacement d'une vertèbre par rapport à une autre entraîne un frottement au niveau de la rotule. Ces frottements peuvent produire, relativement rapidement, une irritation et, après un certain temps, des débris d'usure migrants. Ces débris d'usure peuvent provoquer une réaction à corps étranger qui, si elle se développe au contact du canal médulaire, peut être la cause de troubles neurologiques.

Une seconde technique de réalisation de prothèses entièrement artificielles consiste à utiliser une architecture déformable reliée, rigidement, aux plateaux vertébraux adjacents. Une telle prothèse est décrite, par exemple, dans le document FR-A-2 124 815. Cette prothèse consiste, plus précisément, en un élément affectant la forme d'un disque intervertébral et réalisé dans un matériau élastomère du type silicone. Elle a pour inconvénient de ne pouvoir assurer qu'une mobilité limitée des vertèbres adjacentes, son aptitude à se déformer étant relativement limitée. En outre, cette prothèse n'interdit pas l'expansion radiale du matériau élastomère lors des contraintes exercées sur la prothèse, ce qui se traduit par un risque d'herniation vers le canal médullaire.

Le document FR 2 709 949 concerne une prothèse de disque invertébral comprenant deux demi-enveloppes en forme de coupelles, fixées chacune sur une des deux vertèbres adjacentes au disque vertébral à remplacer, entre lesquelles est disposé un coussin de compression réalisé en au moins deux matériaux, le coussin étant entouré par une ceinture.

Une telle prothèse présente l'inconvénient d'un manque de fiabilité en raison des risques de désolidarisation du coussin et des coupelles sous l'effet des forces de cisaillement qui s'exercent entre le coussin et les coupelles, notamment lorsque la prothèse est en position inclinée par rapport à l'horizontale, en conditions d'utilisation.

Le frottement du coussin sur la ceinture peut générer des particules d'usure qui entraînent des désordres neurologiques. En outre, la désolidarisation du coussin des coupelles entraîne également un risque de déplacement de ce coussin, qui peut rentrer en conflit avec des parties vitales.

Le document FR 2 734 148 décrit une prothèse de disque intervertébral selon le préambule de la revendication 1.

Le problème technique à la base de l'invention est la réalisation d'une prothèse de disque intervertébral qui soit d'une réalisation simple, d'une structure compacte, et qui présente une parfaite sécurité d'utilisation, notamment en évitant des risques de désolidarisation de différentes parties constitutives de la prothèse, notamment sous l'effet de contraintes de cisaillement.

A cet effet, la présente invention concerne une prothèse de disque intervertébral selon la revendication 1.

Cette structure permet à la prothèse de parfaitement résister aux contraintes de cisaillement, puisque celles-ci ne sont plus absorbées, comme tel est le cas habituellement par les seules interfaces entre le coussin de compression et les coupelles, avec risque de désolidarisation subséquent, mais aussi par compression entre le plot central et la paroi de la cheminée. En outre il faut noter qu'en cas de rupture à l'interface entre le coussin et l'une des demi-enveloppes, la sécurité est assurée dans la mesure où le couple plot-cheminée va limiter le déplacement relatif des deux demi-enveloppes rigides.

Avantageusement, les volumes disposés respectivement à l'extérieur et à l'intérieur de la cheminée sont remplis de matériaux compressibles de duretés différentes, le matériau compressible situé à l'extérieur de la cheminée étant plus dur que le matériau situé à l'intérieur de celle-ci. En effet, le matériau situé à l'extérieur de la cheminée doit absorber les efforts de compression dus notamment au poids du corps et les efforts de rotation lors des mouvements du corps. Ce matériau se déforme peu sous des charges fortes. Au contraire, le matériau situé à l'intérieur de la cheminée, possède une dureté inférieure, et est peu compressible en volume, avec une capacité de déformation réduite sous une charge réduite.

Avantageusement, le matériau compressible situé à l'extérieur de la cheminée possède une dureté shore A comprise entre 60 et 100 et de préférence de 80, tandis que le matériau compressible, situé à l'intérieur de la cheminée possède une dureté shore A comprise entre 25 et 30 et de préférence de 28.

Suivant une forme d'exécution de cette prothèse, le matériau compressible situé à l'extérieur de la cheminée est une matière synthétique de type polyuréthanne, et le matériau compressible situé à l'intérieur de la cheminée peut notamment être constitué par un mélange d'élastomère silicone bi-composant, réticulant à température ambiante, et d'un copolymère encapsulant dont l'agent de gonflage est l'isobutane. Il doit être noté que le polyuréthanne possède d'excellentes propriétés de bio-stabilité. Les deux demi-enveloppes ou plateaux sont réalisés en un alliage à base de titane, et comportent chacune sur leur face extérieure des pointes destinées à favoriser leur fixation sur une vertèbre, et sur leur face intérieure des ergots d'accrochage du coussin de compression.

Suivant une caractéristique intéressante de l'invention, le plot faisant saillie d'une demi-enveloppe est fixé par vissage dans un trou traversant que comporte celle-ci.

Un procédé de fabrication de cette prothèse consiste à placer les deux demi-enveloppes dans un moule, le plot étant retiré, à injecter dans le volume extérieur à la cheminée, le matériau de plus grande dureté, à couler dans le volume intérieur de la cheminée le matériau de plus faible dureté par l'ouverture de la demi-enveloppe destinée à recevoir le plot, puis à fixer le plot par vissage.

L'ouverture pratiquée dans la demi-enveloppe extérieure est obturée par le plot qui est fixé par vissage avant la fin de réticulation de l'élastomère, qui assure ainsi une impossibilité de dévissage dans le corps humain. Il doit être noté également que le matériau extérieur adhère au plateau en titane, assurant une étanchéité suffisante pour permettre le coulage du matériau situé à l'intérieur de la cheminée, sans perte de matière.

De toutes façons l'invention sera bien comprise, à l'aide de la description qui suit, en référence aux dessins schématiques annexés représentant, à titre d'exemples non limitatifs, deux formes d'exécution de cette prothèse.
Figure 1 est une vue en perspective d'une portion de colonne vertébrale incluant une prothèse selon l'invention,
Figure 2 est une vue en coupe transversale et à échelle agrandie de cette prothèse selon la ligne II-II figure 3.
Figure 3 est une vue en élévation de cette prothèse.
Figure 4 en est une vue en coupe transversale par un autre plan de coupe.
Figures 5 et 6 sont deux vues de cette prothèse travaillant au cisaillement, dans un cas normal de fonctionnement et dans un cas de rupture d'une interface, respectivement.
Figure 7 est une vue similaire à figure 2, d'une autre prothèse.
Figure 8 est une vue en coupe de cette prothèse, selon la ligne VIII-VIII de figure 7.

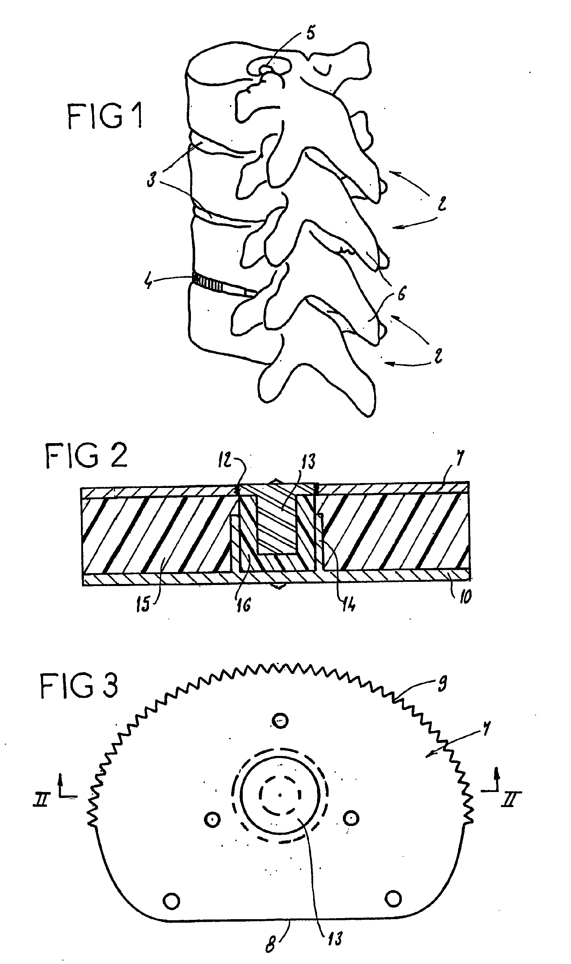

Figure 1 du dessin annexé représente un tronçon de colonne vertébrale, illustrant plus précisément quatre vertèbres 2, qui sont reliées, pour les trois vertèbres du haut par deux disques intervertébraux 3, et pour les deux vertèbres du bas par une prothèse intervertébrale 4. Cette figure montre également le trou vertébral 5 rempli de moelle spinale, ainsi que les apophyses 6.

La figure 3 représente une prothèse intervertébrale en vue de face. Dans cette figure 3, est visible un plateau 7 constitutif de la prothèse, qui est réalisée en alliage de titane, et qui possède un côté 8 sensiblement rectiligne, et un côté 9 courbe comportant une denture. Cette denture sert à faciliter la manipulation de la prothèse par le praticien, lors de la mise en place de celle-ci.

La prothèse est mieux illustrée à la figure 2. Cette prothèse comporte un plateau supérieur 7 et un plateau inférieur 10 réalisés en alliage à base de titane. Le plateau supérieur 7 comporte en son centre, une ouverture taraudée 12 permettant le montage par vissage d'un plot cylindrique fileté 13. Le plateau 10 comporte pour sa part, dans sa zone centrale, une cheminée 14 de section circulaire faisant saillie du coté du plateau 7. La longueur cumulée du plot 13 et de la cheminée 14 est supérieure à la distance entre les deux plateaux 7 et 10. Un coussin de matériau compressible est disposé entre les plateaux 7 et 10. Ce coussin, réalisé en deux parties, comporte un premier matériau 15 disposé à l'extérieur de la cheminée 14, qui est réalisé en un matériau compressible plus dur qu'un matériau compressible 16 disposé à l'intérieur de la cheminée, remplissant l'espace compris entre celle-ci et le plot 13.

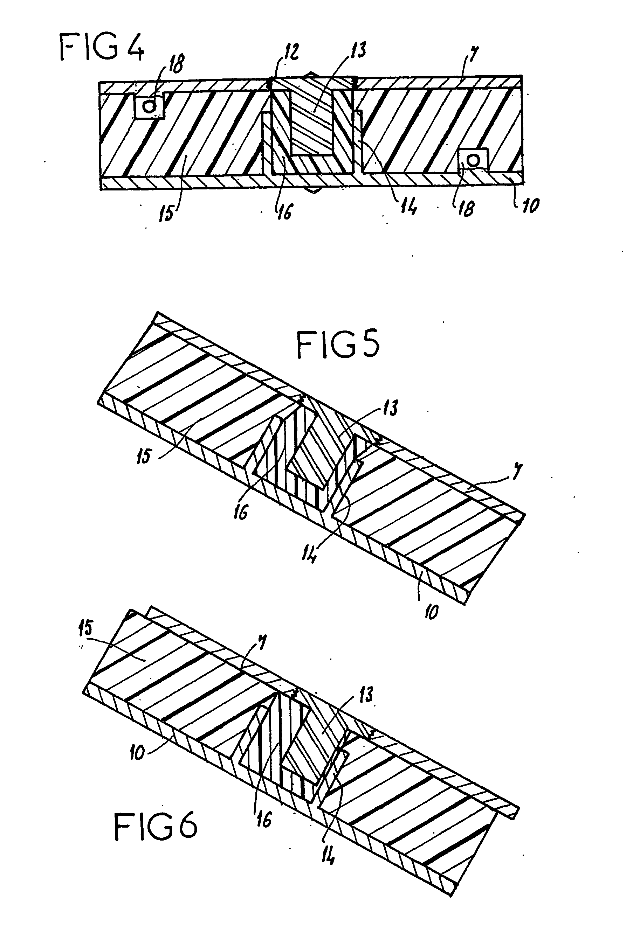

Comme il est visible sur les figures 2 à 4, les plateaux 7 et 10 comportent sur leurs faces extérieures, des pointes, destinées à favoriser la fixation sur les vertèbres contres lesquelles les plateaux sont en contact. En outre, comme montré à la figure 4, les plateaux 7 et 10 comportent, sur leurs faces intérieures, c'est-à-dire sur leur face tournées l'une vers l'autre, des ergots 18 favorisant l'accrochage du matériau amortisseur compressible 15.

Il doit être noté que le matériau compressible 15 peut être constitué par une matière synthétique de type polyuréthanne, tandis que le matériau 16 peut être constitué par un mélange d'élastomère silicone bi-composants, réticulant à température ambiante, et d'un copolymère encapsulant dont l'agent de gonflage est l'isobutane.

Les figures 5 et 6 représentent la prothèse en cours de fonctionnement, et notamment dans le cas d'un fonctionnement incliné, ce qui est habituel en raison de la localisation de certaines prothèses. Il ressort de la figure 5 qu'il existe d'une part un mouvement de cisaillement entre les matériaux compressibles et les plateaux, mais également un phénomène de compression du matériau 16 entre la cheminée 14 et le plot 13. Cet effet de compression permet de limiter les effets du cisaillement, et notamment d'éviter une désolidarisation entre le matériau compressible 15 et l'un ou l'autre des plateaux.

La figure 6 représente le cas extrême de la rupture de l'interface entre le matériau compressible 15 et le plateau supérieur 7. Il est intéressant de constater que même dans cette hypothèse extrême, il n'y a pas de désolidarisation totale des différents composants puisque le plot 13 vient prendre appui contre la cheminée 14, avec interposition d'une certaine masse de matière, ce qui évite tout risque pour le patient.

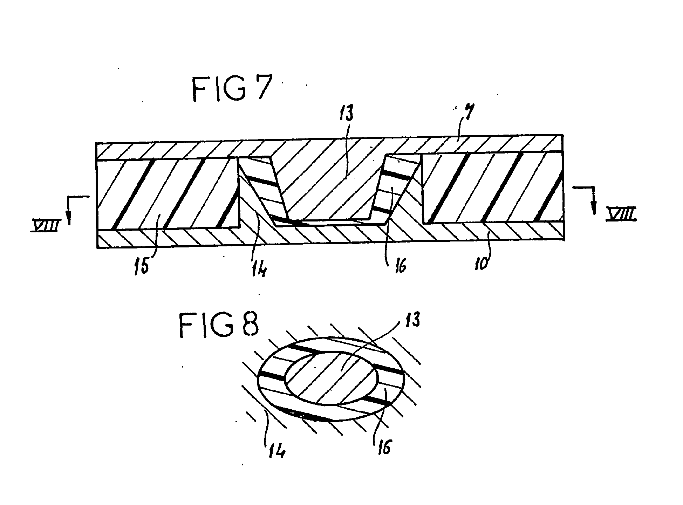

Les figures 7 et 8 représentent une variante d'exécution de cette prothèse, dans laquelle les mêmes éléments sont désignés par les mêmes références que précédemment. Dans ce cas, le plot 13 et la cheminée 14 présentent une section trapézoïdale, en coupe longitudinale, la section du plot diminuant du côté de son extrémité libre. En outre, vus en coupe transversale, le plot 13 et la cheminée 14 ont une section non circulaire, par exemple en forme d'ellipse, afin d'empêcher une rotation relative des deux demi-enveloppes (7, 10) ou du matériau compressible (15).

Dans tous les cas, il est avantageux que les surfaces externes des demi-enveloppes comportent un revêtement d'hydroxyapatite ou par création de micro-porosités.

Comme il ressort de ce qui précède l'invention apporte une grande amélioration à la technique existante, en fournissant une prothèse intervertébrale de structure simple et compacte , qui peut être, compte tenu de sa structure, réalisée avec les matériaux et notamment avec des plateaux d'épaisseur réduite, tout en possédant une excellente solidité et une excellente fiabilité.

Comme il va de soi l'invention ne se limite pas aux seules formes d'exécution de cette prothèse intervertébrale, décrites ci-dessus à titre d'exemples, elle en embrasse au contraire toutes les variantes. C'est ainsi, notamment que les matériaux compressibles pourraient être différents, que le plot du plateau supérieur pourrait ne pas être amovible, ou encore que les plateaux pourraient ne pas posséder une forme plane mais posséder une forme de coupelle, sans que l'on sorte pour autant du cadre de l'invention.

## Revendications

1. Prothèse de disque intervertébral comportant deux demi-enveloppes rigides en forme de coupelles ou de plateaux (7,10), destinées chacune à tre fixées sur une des deux vertèbres adjacentes au disque intervertébral à remplacer, les deux demi-enveloppes enserrant un coussin de compression réalisé en au moins deux matériaux de duretés différentes, l'une (10) des deux demi-enveloppes comportant dans sa zone centrale une cheminée (14) tournée vers l'autre demi- enveloppe (7), la seconde demi-enveloppe (7) comportant dans sa zone centrale un plot (13) de section inférieure à celle de la cheminée, tourné vers la première demi-enveloppe et engagé dans la cheminée de celle-ci, la somme des longueurs de la cheminée (14) et du plot (13) étant supérieure à la distance entre les deux demi-enveloppes (7,10), **caractérisée en ce que** le coussin de compression comporte une première partie (16) disposée à l'intérieur de la cheminée (14) et remplissant l'espace compris entre la cheminée et le plot (13), et une deuxième partie disposée à l'extérieur de la cheminée (14), la première partie (16) étant réalisée en un premier matériau compressible et la deuxième partie (15) étant réalisée en un deuxième matériau compressible plus dur que le premier matériau compressible.

2. Prothèse selon la revendication 1, **caractérisée en ce que** le deuxième matériau compressible (15) situé à l'extérieur de la cheminée (14) possède une dureté shore A comprise entre 60 et 100 et de préférence de 80, tandis que le premier matériau compressible (16), situé à l'intérieur de la cheminée (14) possède une dureté shore A comprise entre 25 et 30 et de préférence de 28.

3. Prothèse selon l'une des revendications 1 et 2, **caractérisée en ce que** le deuxième matériau compressible (15) situé à l'extérieur de la cheminée est une matière synthétique de type polycarbonate uréthane.

4. Prothèse selon l'une des revendications 1 et 3, **caractérisé en ce que** le premier matériau compressible (16) situé à l'intérieur de la cheminée est constitué par un mélange d'élastomère silicone bi-composant, réticulant à température ambiante, et d'un copolymère encapsulant dont l'agent de gonflage est l'isobutane.

5. Prothèse selon l'une des revendications 1 à 4, **caractérisée en ce que** les deux demi-enveloppes (7, 10) sont réalisées en un alliage à base de titane.

6. Prothèse selon l'une des revendications 1 à 5, **caractérisée en ce que** chaque demi-enveloppe (7,10) comporte, sur sa face extérieure, des pointes (17) destinées à favoriser sa fixation sur une vertèbre (2).

7. Prothèse selon l'une des revendications 1 à 6, **caractérisée en ce que** chaque demi-enveloppe (7,10) comporte, sur sa face intérieure, des ergots (18) d'accrochage du coussin de compression (15).

8. Prothèse selon l'une des revendications 1 à 7, **caractérisée en ce que** le plot (13) faisant saillie d'une demi-enveloppe (7) est fixé par vissage dans un trou (12) traversant que comporte celle-ci.

9. Prothèse selon l'une des revendications 1 à 8, **caractérisé en ce que** le plot (13) et la cheminée (14) possèdent une section trapézoïdale.

10. Prothèse selon l'une des revendications 1 à 9, **caractérisé en ce que** le plot (13) et la cheminée (14) possèdent, en coupe transversale, une section non circulaire.

11. Prothèse selon l'une des revendications 1 à 10, **caractérisé en ce que** les surfaces externes des demi-enveloppes (7,10) comportent un revêtement destiné à assurer une fixation osseuse primaire, tel qu'un revêtement d'hydroxyapatite ou des micro-porosités.

12. Procédé de fabrication d'une prothèse selon l'une des revendication 1 à 3 et 8 à 11, **caractérisé en ce qu'**il consiste à placer les deux demi-enveloppes dans un moule, le plot (13) étant retiré, à injecter dans le volume extérieur à la cheminée (14), le deuxième matériau (15) de plus grande dureté, à couler dans le volume intérieur de la cheminée (14) le premier matériau (16) de plus faible dureté par l'ouverture (12) de la demi-enveloppe (7) destinée à recevoir le plot (13), puis à fixer le plot (13) par vissage.

## Claims

1. An intervertebral disc prosthesis comprising two rigid half-enclosures in the shape of cups or plates (7, 10), each designed to be fastened on one of the two vertebrae adjacent to the intervertebral disc to be replaced, the two half-enclosures gripping a compression cushion made from at least two materials of different hardnesses, one (10) of the two half-enclosures comprising a funnel (14) in its central area turned toward the other half-enclosure (7), the second half-enclosure (7) comprising a stud (13) in its central area with a section smaller than that of the funnel, turned toward the first half-enclosure and engaged in the funnel thereof, the sum of the lengths of the funnel (14) and the stud (13) being larger than the distance between the two half-enclosures (7, 10), **characterized in that** the compression cushion comprises a first portion (16) arranged inside the funnel (14) and filling the space between the funnel and the stud (13), and a second portion arranged outside the funnel (14), the first portion (16) being made from a first compressible material and the second portion (15) being made from a second compressible material that is harder than the first compressible material.

2. The prosthesis according to claim 1, **characterized in that** the second compressible material (15) situated outside the funnel (14) has a Shore A hardness between 60 and 100 and preferably 80, while the first compressible material (16), situated inside the funnel (14), has a Shore A hardness between 25 and 30, and preferably 28.

3. The prosthesis according to one of claims 1 and 2, **characterized in that** the second compressible material (15) situated outside the funnel is a synthetic material of the polycarbonate urethane type.

4. The prosthesis according to one of claims 1 to 3, **characterized in that** the first compressible material (16) situated inside the funnel is made up of a bicomponent silicon elastomer mixture, cross-linking at ambient temperature, and an encapsulating copolymer whereof the foaming agent is isobutene.

5. The prosthesis according to one of claims 1 to 4, **characterized in that** the two half-enclosures (7, 10) are made from a titanium-based alloy.

6. The prosthesis according to one of claims 1 to 5, **characterized in that** each half-enclosure (7, 10) comprises spikes (17) on its outer surface that are designed to favor its fastening on a vertebra (2).

7. The prosthesis according to one of claims 1 to 6, **characterized in that** each half-enclosure (7, 10) comprises lugs (18) on its inner surface for attaching the compression cushion (15).

8. The prosthesis according to one of claims 1 to 7, **characterized in that** the stud (13) protruding from a half-enclosure (7) is fastened by screwing in a through hole (12) comprised by the latter.

9. The prosthesis according to one of claims 1 to 8, **characterized in that** the stud (13) and the funnel (14) have a trapezoidal section.

10. The prosthesis according to one of claims 1 to 9, **characterized in that** the stud (13) and the funnel (14) have a non-circular section in transverse cross-section.

11. The prosthesis according to one of claims 1 to 10, **characterized in that** the outer surfaces of the half-enclosures (7, 10) comprise a covering designed to ensure primary bone fastening, such as a hydroxyapatite covering or micro-porosities.

12. A method for manufacturing a prosthesis according to one of claims 1 to 3 and 8 to 11, **characterized in that** it consists of placing the two half-enclosures in a mold, the stud (13) being removed, injecting the second (15), harder material into the volume outside the funnel (14), pouring the first, less hard material (16) into the inner volume of the funnel (14) through the opening (12) of the half-enclosure (7) designed to receive the stud (13), then fastening the stud (13) by screwing.

## Patentansprüche

1. Bandscheibenprothese, die zwei starre Halbhüllen in Form von Schalen oder Platten (7, 10) aufweist, die jeweils dazu bestimmt sind, auf einem der zwei benachbarten Wirbel der zu ersetzenden Bandscheibe befestigt zu sein, wobei die zwei Halbhüllen ein Kompressionskissen umschließen, das aus mindestens zwei Materialen unterschiedlicher Härten hergestellt ist, wobei eine (10) der zwei Halbhüllen in ihrer zentralen Zone einen zu der anderen Halbhülle (7) gedrehten Schacht (14) aufweist, wobei die zweite Halbhülle (7) in ihrer zentralen Zone einen Stift (13) mit einem kleineren Querschnitt als der des Schachts aufweist, der zur ersten Halbhülle zeigt und in den Schacht derselben eingreift, wobei die Summe der Längen des Schachts (14) und des Stifts (13) größer ist als der Abstand zwischen den zwei Halbhüllen (7, 10), **dadurch gekennzeichnet, dass** das Kompressionskissen einen ersten Abschnitt (16) aufweist, der im Schacht (14) angeordnet ist und den Raum zwischen dem Schacht und dem Stift (13) füllt, und einen zweiten Abschnitt, der außerhalb des Schachts (14) angeordnet ist, wobei der erste Abschnitt (16) aus einem ersten komprimierbaren Material und der zweite Abschnitt (15) aus einem zweiten komprimierbaren Material hergestellt ist, das härter ist als das erste komprimierbare Material.

2. Prothese nach Anspruch 1, **dadurch gekennzeichnet, dass** das zweite komprimierbare Material (15), das sich außerhalb des Schachts (14) befindet, eine Shore-Härte A zwischen 60 und 100 inklusive und vorzugsweise von 80 besitzt, wogegen das erste komprimierbare Material (16), das sich im Schacht (14) befindet, eine Shore-Härte A zwischen 25 und 30 inklusive und vorzugsweise von 28 besitzt.

3. Prothese nach einem der Ansprüche 1 und 2, **dadurch gekennzeichnet, dass** das zweite komprimierbare Material (15), das sich außerhalb des Schachts befindet, ein synthetisches Material vom Typ Polycarbonaturethan ist.

4. Prothese nach einem der Ansprüche 1 und 3, **dadurch gekennzeichnet, dass** das erste komprimierbare Material (16), das sich im Schacht befindet, aus einem Zweikomponenten-Elastomersilikongemisch besteht, das bei Raumtemperatur vernetzt, und einem einkapselnden Copolymer, dessen Treibmittel das Isobutan ist.

5. Prothese nach einem der Ansprüche 1 bis 4, **dadurch gekennzeichnet, dass** die zwei Halbhüllen (7, 10) aus einer Legierung auf der Basis von Titan hergestellt sind.

6. Prothese nach einem der Ansprüche 1 bis 5, **dadurch gekennzeichnet, dass** jede Halbhülle (7, 10) auf ihrer Außenseite Spitzen (17) aufweist, die dazu bestimmt sind, die Fixierung auf einem Wirbel (2) zu unterstützen.

7. Prothese nach einem der Ansprüche 1 bis 6, **dadurch gekennzeichnet, dass** jede Halbhülle (7,10) auf ihrer Innenseite Fixierzapfen (18) des Kompressionskissens (15) aufweist.

8. Prothese nach einem der Ansprüche 1 bis 7, **dadurch gekennzeichnet, dass** der aus einer Halbhülle (7) herausragende Stift (13) durch Schrauben in einem durchgehenden Loch (12) befestigt ist, das diese aufweist.

9. Prothese nach einem der Ansprüche 1 bis 8, **dadurch gekennzeichnet, dass** der Stift (13) und der Schacht (14) eine trapezförmigen Durchmesser besitzen.

10. Prothese nach einem der Ansprüche 1 bis 9, **dadurch gekennzeichnet, dass** der Stift (13) und der Schacht (14) im Querschnitt einen nicht kreisrunden Durchmesser besitzen.

11. Prothese nach einem der Ansprüche 1 bis 10, **dadurch gekennzeichnet, dass** die Außenflächen der Halbhüllen (7, 10) eine Beschichtung aufweisen, die dazu bestimmt ist, eine primäre Knochenfixierung sicherzustellen, wie eine Hydroxyapatitbeschichtung, oder Mikroporositäten.

12. Herstellungsverfahren einer Prothese nach einem der Ansprüche 1 bis 3 und 8 bis 11, **dadurch gekennzeichnet, dass** es darin besteht, die zwei Halbhüllen in einer Form zu platzieren, wobei der Stift (13) herausgezogen ist, in das Volumen außerhalb des Schachts (14) das zweite Material (15) höherer Härte einzuspritzen, in das Volumen im Schacht (14) das erste Material (16) geringerer Härte durch die Öffnung (12) der Halbhülle (7) zu gießen, die dazu bestimmt ist, den Stift (13) aufzunehmen und dann den Stift (13) durch Schrauben zu fixieren.
